# EUROPEAN PATENT APPLICATION

(11) **EP 1 975 155 A1**
(43) Date of publication of application: **01.10.2008**
(21) Application number: 06832839.2
(22) Date of filing: 17.11.2006
(51) Int. Cl.: C07D 201/08, C07D 225/02, C07B 61/00

(54) **PROCESS FOR PRODUCTION OF LACTAM**

(30) Priority: 07.12.2005 JP 2005353205
(71) Applicant: Ube Industries, Ltd., Ube-shi Yamaguchi 755-8633 (JP)
(72) Inventor: KUGIMOTO, Junichi, Ube-shi Yamaguchi 755-8633 (JP)
(74) Representative: Bublak, Wolfgang
(86) International application number: PCT/JP2006/322960
(87) International publication number: WO 2007/066492

(57) **Abstract**

Disclosed is a process for production of a lactam (particularly, an industrially useful lactam, e.g., laurolactam) with high selectivity, comprising hydrogenating a cyclic dicarboxylic acid imide having a large of ring members (particularly, a 8-membered or more cyclic dicarboxylic acid imide) to produce a corresponding. The process is **characterized by** hydrogenating a 8-membered or more cyclic dicarboxylic acid imide in the presence of ruthenium of platinum. In the process, the ruthenium or platinum may be supported on activated charcoal and an alcohol may be used as a reaction solvent.

## Description

### TECHNICAL FIELD

The present invention relates to a process for production of a lactam by selectively hydrogenating one carbonyl group in a cyclic dicarboxylic acid imide.

### BACKGROUND ART

Industrial lactam producing processes include a process comprising oximating a cyclic ketone and then bringing the resultant oxime in Beckmann rearrangement, as well known. Most of caprolactam and laurolactam, the major part of lactam products, are produced through this process. This process, however, uses a large quantity of a corrosive concentrated sulfuric acid or a fuming sulfuric acid and discharges a large quantity of ammonium sulfate. Accordingly, it is a process with heavy environmental impact. In contrast, a lactam producing process with hydrogenation of a cyclic dicarboxylic acid imide stoichiometrically only yields water as a by-product equimolar to the lactam. Accordingly, it is expected to reduce the environmental impact.

As the lactam producing process with hydrogenation of a cyclic dicarboxylic acid imide, Patent Document 1 proposes a process for hydrogenating a C₄₋₆ cyclic dicarboxylic acid imide or an N-substituted form thereof (such as N-alkylated form) in gaseous phase. This process uses a catalyst that contains copper as an essential component (such as copper-chromium, and copper-zinc). This process is substantially suitable for producing a small ring lactam corresponding to a C₄ cyclic dicarboxylic acid imide such as a succinic acid and a maleic acid. In fact, when the inventor of the present application applied this process to a C₁₂ cyclic dicarboxylic acid imide, no laurolactam could be produced.

Patent Document 2 discloses a process for hydrogenating a cyclic dicarboxylic acid imide having a C₂ or more divalent organic group between carbonyl groups or an N-substituted form thereof (such as N-alkylated form) in liquid phase. This process uses a catalyst that contains cobalt or nickel as the principal component, preferably a catalyst that contains cobalt as the principal component and a decorative component of at least one metal component of molybdenum, tungsten and rhenium, and adds an acidic substance to the reaction system. The divalent organic group is an organic residue composed of a dibasic acid. Examples of the dibasic acid include a maleic acid, a succinic acid, a glutaric acid, an adipic acid and a phthalic acid. This process is substantially suitable for producing a small ring lactam, like the above process.

Patent Document 3 discloses a process for producing 2-pyrolidone from a maleic anhydride, hydrogen, and ammonia or primary amine (process for hydrogenating a 5-membered cyclic dicarboxylic acid to produce a corresponding lactam). This process uses a hydrogenation catalyst (such as Raney nickel, Raney cobalt, palladium, and platinum). It fails, however, to disclose any process for hydrogenating a cyclic dicarboxylic acid imide having a large number of ring members to produce a corresponding lactam.

Patent Document 1: JP 63-27476A
Patent Document 2: JP 63-27474A
Patent Document 3: US Patent No. 3109005

### DISCLOSURE OF THE INVENTION

### PROBLEM TO BE SOLVED BY THE INVENTION

As described above, there has been not known any example of hydrogenation of a cyclic dicarboxylic acid imide having a large number of ring members to produce a corresponding lactam with higher selectivity. The present invention has been made in consideration of such the situation. It has a subject to provide a process capable of hydrogenating a cyclic dicarboxylic acid imide having a large number of ring members (particularly, an 8-membered or more cyclic dicarboxylic acid imide) to produce a corresponding lactam (particularly, industrially useful lactam such as laurolactam) with higher selectivity.

### MEANS TO SOLVE THE PROBLEM

The inventor et al. eagerly studied and consequently found that hydrogenation of an 8-membered or more cyclic dicarboxylic acid imide with a catalyst of ruthenium or platinum makes it possible to produce a corresponding lactam with higher selectivity, and finally reached completion of the invention.

The subject of the present invention can be solved by the following invention.
(1) A process for production of a lactam, comprising hydrogenating an 8-membered or more cyclic dicarboxylic acid imide in the presence of ruthenium or platinum.
(2) A process for production of a lactam according to the first invention, wherein an 8- to 25-membered cyclic dicarboxylic acid imide is used as the cyclic dicarboxylic acid imide.
(3) A process for production of a lactam according to the first invention, wherein the ruthenium or platinum is supported on active charcoal for use.
(4) A process for production of a lactam according to any one of the first through the third inventions, wherein an alcohol is used as a reaction solvent.
(5) A process for production of a lactam according to the fourth invention, wherein a t-butyl alcohol is used as the alcohol.

### EFFECT OF THE INVENTION

The present invention is capable of hydrogenating a cyclic dicarboxylic acid imide having a large number of ring members (particularly, an 8-membered or more cyclic dicarboxylic acid imide) to produce a corresponding lactam (particularly, industrially useful lactam such as laurolactam) with higher selectivity. It is also possible by the present invention to efficiently produce a lactam having a large number of ring members through a simple process without generation of a large quantity of by-products with heavy environmental impact.

Namely, a general process for producing a lactam having a large number of ring members requires massive incidental facilities in oximation of a cyclic ketone and Beckmann rearrangement of the resultant oxime. For example, it requires production facilities for a sulfuric acid and a fuming sulfuric acid, production facilities for hydroxylamine, and treatment facilities for by-product ammonium sulfate, which extremely increase the environmental impact. In contrast, the lactam producing process with hydrogenation of a cyclic dicarboxylic acid imide of the present invention only yields water as a by-product equimolar to the lactam. Accordingly, it is possible to reduce the environmental impact extremely even taking material productions into account. For example, in production of laurolactam, the cyclic dicarboxylic acid imide of the material of laurolactam can be easily produced through thermal decomposition of 1,1'-peroxy dicyclohexylamine made of cyclohexanone, hydrogen peroxide and ammonia. Other incidental facilities just require production facilities for 1,1'-peroxy dicyclohexylamine and production facilities for hydrogen peroxide. Ammonia can be used as it is without transformation to hydroxylamine.

### THE BEST MODE FOR CARRYING OUT THE INVENTION

The present invention will now be described in detail.
The cyclic dicarboxylic acid imide used in the present invention includes a cyclic dicarboxylic acid imide having a large number of ring members, that is, an 8-membered or more cyclic dicarboxylic acid imide. Among those, preferably an 8-to 25-membered, more preferably an 11- to 17-membered, and particularly preferably an 11- to 15-membered cyclic dicarboxylic acid imide is used. Examples of the cyclic dicarboxylic acid imide include decane-1,10-dicarbonimide; nonane-1,9-dicarbonimide; octane-1,8-dicarbonimide; and hexane-1,6-dicarbonimide.

The above cyclic dicarboxylic acid imide can be produced through publicly known methods. For example, decane-1,10-dicarbonimide can be produced easily by reacting cyclohexanone, hydrogen peroxide and ammonia together, and then thermally decomposing the resultant 1,1'-peroxy-dicyclohexylamine. Other cyclic dicarboxylic acid imide can be similarly produced from corresponding cyclic ketone, hydrogen peroxide and ammonia.

The present invention uses a hydrogenation catalyst of ruthenium or platinum. Ruthenium and platinum have no restriction on the oxidized state and the shape though a 0-valent metal is suitable. Ruthenium or platinum may be supported on a support for use (as a supported catalyst). An appropriate support in this case is active carbon.

The usage of the hydrogenation catalyst varies in accordance with the form of a reactor though there is no harm if it can be economical from the viewpoint of reaction rates and catalyst costs. For example, in the case of a batch reactor (suspended bed), the usage is preferably 0.00001-1 mol, and more preferably 0.001-0.5 mol on the basis of metal conversion relative to 1 mol of a cyclic dicarboxylic acid imide.

There is no restriction on a support method in preparation of the supported catalyst. For example, a publicly known method such as an adsorption method, a pore filling method, an incipient wetness method, an evaporation-dryness method, and a spray method may be used to support a compound of ruthenium or platinum on a support. The compound is then reduced to a 0-valent metal with a reducing agent such as hydrogen. The reduction to the 0-valent metal may be performed in a reactor before the initiation of a reaction. There is no harm in using a commercially available supported catalyst. The quantity of the metal supported in the supported catalyst is preferably 0.01-20 weight %, more preferably 0.1-10 weight % relative to the support.

Examples of the above ruthenium compound include ruthenium chloride, ruthenium bromide, ruthenium iodide, ruthenium nitrosylchloride, ruthenium di-chlorotris (triphenylphosphine), and ruthenium acetylacetonate. Examples of the platinum compound include platinum chloride, platinum bromide, platinum iodide, platinum cyanide, platinum acetylacetonate, and a platinum-diamine-dichloro complex. These ruthenium or platinum compounds may be used in a hydrogenation reaction as they are, that is, not supported on a support.

In the hydrogenation reaction, hydrogen is used solely or diluted with an inert gas such as nitrogen. Suitable partial pressure of hydrogen varies in accordance with the form of a reaction though there is no harm if it can be economical from the viewpoint of reaction rates and equipment costs and the selectivity can be kept high. For example, in the case of a batch reactor (suspended bed), the partial pressure is preferably 0.01-50 MPa, more preferably 1-20 MPa. The temperature of the hydrogenation reaction is preferably 50-250 °C, more preferably 70-200 °C.

In the hydrogenation reaction, as a cyclic dicarboxylic acid imide generally has a higher melting point, the use of a reaction solvent is preferable. There is no particular restriction on the reaction solvent if the reaction solvent itself can not be hydrogenated and can not interfere with the reaction. For example, available reaction solvents include chain saturated hydrocarbons (such as n-hexane, and isooctane); cyclic saturated hydrocarbons (such as cyclohexane, cyclooctane, methyl cyclohexane, and isopropyl cyclohexane); ethers (such as tetrahydrofuran); carboxylic esters (such as ethyl acetate, butyl acetate, and caprolactone); carbonic esters (such as dimethyl carbonate); alcohol (such as methyl alcohol, ethyl alcohol, isopropyl alcohol, t-butyl alcohol, and ethylene glycol); amides (such as N-methyl pyrrolidone, and ε-caprolactam); and water. These reaction solvents are preferable if they can provide a higher solubility of the cyclic dicarboxylic acid imide. Specifically, alcohol, especially t-butyl alcohol is most preferable. The reaction solvent is used, in accordance with the type, if it does not lose the solubility of the cyclic dicarboxylic acid imide and the stability, and it can be economical from the viewpoint of reaction rates and solvent costs.

In hydrogenation reactions, any types of reactors usually available in industrial productions, such as a batch reactor, a semi-batch reactor, and a flow reactor, may be used without trouble if they can execute a hydrogenation reaction of a cyclic dicarboxylic acid imide. If a supported catalyst is used, there is no harm in being applied any one of a suspended bed, a fixed bed, a fluidized bed, and a movable bed if it can execute a hydrogenation reaction. After the reaction, the produced lactam is separated and purified through a usual method such as distillation and crystallization.

### EXAMPLES

The present invention is specifically described next with Examples and Comparative examples. Hereinafter, PXA represents 1,1'-peroxy-dicyclohexylamine. 5%Ru/C represents a ruthenium supported catalyst with a support of active charcoal, which supports 5 weight % ruthenium. 5%Pt/C represents a platinum supported catalyst with a support of active charcoal, which supports 5 weight % platinum. 5%Pd/C represents a palladium supported catalyst with a support of active carbon, which supports 5 weight % palladium.

### [Referential example 1: Production of PXA]

100g of cylcohexanone, 30g of 60 weight % hydrogen peroxide, 130g of 20 weight % aqueous ammonia, and 15g of ammonium chloride were fed into a reactor of the agitation tank type, and subjected to a reaction at 30 °C for 9 hours to obtain 190g of crude crystals of PXA. As a result of a gas chromatography analysis, the cylcohexanone conversion was 90 % and the PXA selectivity was 95 %. The rough crystals were recrystallized (with a solvent: n-hexane) to obtain 110g of PXA.

### [Referential example 2: Production of Decane-1,10-dicarbonimide]

54g of the resultant PXA was dissolved in 560 ml of toluene and the liquid was sent to a tube-type reactor having an inside diameter of 0.5 mm heated at 270 °C to thermally decompose PXA. The reaction pressure at this time is 10 MPa-G and the average residence time of the reaction liquid in the reactor was 6 seconds. As a result of a gas chromatography analysis, the PXA conversion was 100 %, and products of decane-1,10-dicarbonimide and cylcohexanone were confirmed. After distillation removal of toluene and cylcohexanone, recrystallization (with a solvent: acetonitrile) was performed to obtain 26.5g of decane-1,10-dicarbonimide.

### [Example 1]

1g of the decane-1,10-dicarbonimide obtained in Referential example 2 was dissolved in 20g of t-butyl alcohol. 4g of a 5%Ru/C powder (from N.E. CHEMCAT) was added thereto. Hydrogen was then injected therein under pressure to achieve 8 MPaG, and finally hydrogenation was performed at 100 °C for 30 minutes. As a result of a gas chromatography analysis, no decane-1,10-dicarbonimide was present and laurolactam was produced at a selectivity of 90 %.

### [Comparative example 1]

Except that Ru/C was replaced with 0.5 g of a copper-zinc catalyst powder (from Toyo CCI) which consists of copper oxide and zinc oxide and that the reaction time was changed to 3 hours, hydrogenation was performed as in Example 1. As a result, the decane-1,10-dicarbonimide conversion was 84 % and no laurolactam could be detected.

### [Comparative example 2]

Except that Ru/C was replaced with 1 g of 5%Pd/C (from N.E. CHEMCAT) and that the reaction time was changed to 2 hours, hydrogenation was performed as in Example 1. As a result, the decane-1,10-dicarbonimide conversion was 5 % and no laurolactam could be detected.

### [Example 2]

Except that t-butanol was replaced with 10 g of isopropyl alcohol, that the usage of Ru/C was changed to 0.5 g, and that the reaction time was changed to 2 hours, hydrogenation was performed as in Example 1. As a result, no decane-1,10-dicarbonimide was present and laurolactam was produced at a selectivity of 56%.

### [Example 3]

Except that the usage of t-butyl alcohol was changed to 10 g, that Ru/C was replaced with 1 g of a 5%Pt/C powder (from N.E. CHEMCAT), and that the reaction time was changed to 3 hours, hydrogenation was performed as in Example 1. As a result, the decane-1,10-dicarbonimide conversion was 65 % and laurolactam was produced at a selectivity of 72 %.

### [Comparative example 3]

Except that 1g of decane-1,10-dicarbonimide was replaced with 0.5 g of succinimide, hydrogenation was performed as in Example 1. As a result, the succinimide conversion was 100 % and the 2-pyrolidone selectivity was 41 %.

### [Example 4]

Except that Ru/C was replaced with 0.2 g of ruthenium dichloro-tris(triphenylphosphine), and that the reaction time was changed to 3 hours, hydrogenation was performed as in Example 1. As a result, the decane-1,10-dicarbonimide conversion was 82 % and laurolactam was produced at a selectivity of 68 %.

The results from Examples and Comparative examples are shown in Table 1. In the table, the number of ring members represents the number of ring members of a cyclic dicarboxylic acid imide, Cu-Zn represents a copper-zinc catalyst, RuCl₂(PPh₃)₃ represents ruthenium dichlorotris(triphenyl phosphine), t-BuOH represents t-butyl alcohol, and i-PrOH represents isopropyl alcohol.

**[Table 1]**

| | (1) | (2) | (3) | (4) | (5) | (6) |
|---|---|---|---|---|---|---|
| Example 1 | 13 | Ru/C: 4g | t-BuOH | 0.5 | 100 | 90 |
| Comparative example 1 | 13 | Cu-Zn:0.5g | t-BuOH | 3 | 84 | 0 |
| Comparative example 2 | 13 | Pd/C: 1g | t-BuOH | 2 | 5 | 0 |
| Example 2 | 13 | Ru/C:0.5g | i-PrOH | 2 | 100 | 56 |
| Example 3 | 13 | Pt/C: 1g | t-BuOH | 3 | 65 | 72 |
| Comparative example 3 | 4 | Ru/C: 1g | t-BuOH | 0.5 | 100 | 41 |
| Example 4 | 13 | RuCl₂(PPh₃)₃: 0.5g | t-BuOH | 3 | 82 | 68 |

| | | | | | | |
|---|---|---|---|---|---|---|
| (1) Number of Ring Members (2) Catalyst (3) Solvent (4) Reaction Time (h) (5) Conversion (%) (6) Selectivity (%) | | | | | | |

### INDUSTRIAL AVAILABILITY

The present invention is capable of hydrogenating a cyclic dicarboxylic acid imide having a large number of ring members, that is, an 8-membered or more cyclic dicarboxylic acid imide, thereby producing a corresponding lactam with higher selectivity and efficiency and extremely reduced environmental impact. The resultant lactam is a useful substance available in solvents, intermediate materials of various chemical products, and raw material monomers of polyamide.

## Claims

1. A process for production of a lactam, comprising hydrogenating an 8membered or more cyclic dicarboxylic acid imide in the presence of ruthenium or platinum.

2. The process for production of a lactam according to claim 1, wherein an 8-to 25-membered cyclic dicarboxylic acid imide is used as the cyclic dicarboxylic acid imide.

3. The process for production of a lactam according to claim 1, wherein the ruthenium or platinum is supported on active charcoal for use.

4. The process for production of a lactam according to any one of claims 1-3, wherein an alcohol is used as a reaction solvent.

5. The process for production of a lactam according to claim 4, wherein a t-butyl alcohol is used as the alcohol.
